# EUROPEAN PATENT APPLICATION

(11) **EP 2 594 592 A1**
(43) Date of publication of application: **22.05.2013**
(21) Application number: 11806795.8
(22) Date of filing: 12.07.2011
(51) Int. Cl.: C08B 37/00, A61K 31/7125, A61K 47/36, A61K 47/48, A61K 48/00, C07H 21/04, C12N 15/113

(54) **(NUCLEIC ACID)-POLYSACCHARIDE COMPLEX**

(30) Priority: 16.07.2010 JP 2010162039
(71) Applicant: Napajen Pharma, Inc., Burlingame, California 94010 (US)
(72) Inventor: Sakurai, Kazuo, Himeji-shi, Hyogo 670-0033 (JP)
(74) Representative: Müller-Boré & Partner Patentanwälte
(86) International application number: PCT/JP2011/065908
(87) International publication number: WO 2012/008465

(57) **Abstract**

The present invention provides a nucleic acid-polysaccharide complex that is highly stable and has reduced cytotoxicity in a high yield. That is, the present invention provides a nucleic acid-polysaccharide complex of an antisense DNA having polydeoxyadenine (dA) as a polysaccharide binding site and a polysaccharide having a ß-1,3-glucan skeleton wherein at least part of phosphodiester links of the polydeoxyadenine is phosphorothioated (provided that the phosphodiester links of the antisense DNA and the polydeoxyadenine are not entirely phosphorothioated).

## Description

### [Technical Field]

The present invention relates to a highly stable, low-toxicity nucleic acid-polysaccharide complex and a production method therefor.

### [Background Art]

The concept of antisense therapy was proposed by Zamecnik and Stephenson in the 1970s. They found for the first time that a short oligonucleotide sequence that is complementary to a specific portion of the target mRNA can inhibit expression of the corresponding protein (see Non Patent Literature 1). An oligonucleotide that has such a biological function is used as an antisense oligonucleotide (ASO), and currently a large number ofASOs are at stages of preclinical and clinical trials in the areas of HIV/AIDS and cancer studies. It is known from previous research that poor cell transfer ofASOs into a cell and their vulnerability to nuclease significantly deteriorate the *in vivo* activity. In some cases, in order to achieve sufficient biological effects, tests in which an ASO is administered in excessive amounts of 10 mg/kg or greater to animal models are carried out in *in vivo* tests. However, excessive amounts of ASO may cause toxicity and side effects, and are not practical from the commercial and medical viewpoints in the first place. In order to overcome such disadvantages, two main approaches, i.e., nucleic acid analog and target delivery, were proposed.

It is known that among various nucleic acid analogs, an ASO in which the phosphodiester link portion is phosphorothioated (S-modified) is superior in nuclease resistance and cellular uptake. In the phosphorothioated ASO, an oxygen atom of the phosphodiester (PO) group is replaced with a sulfur atom.

It is known that schizophyllan (SPG) on the other hand, which is a natural polysaccharide used for target delivery, is recognized by dectin-1 of an antigen presenting cell, and can be used as a delivery carrier. SPG belongs to the ß-1,3-glucan family, adopts a right-handed triple helix conformation in a neutral aqueous solution, and adopts a single strand conformation in an alkali solution (aqueous sodium hydroxide having 0.25 N or greater). When an alkali solution of SPG is neutralized so as to be at pH 7 to 8, the single strands reproduce the original triple helix conformation by hydrophobic and hydrophilic binding interactions. If there is a specific polynucleotide (for example, a nucleic acid homopolymer such as polydeoxyadenine) is present in the neutralization process, a complex composed of two SPG chain and one polynucleotide chain is formed. Based on this technology, a technology to use ß-1,3-glucan such as SPG has been actively researched. For example, so far, a technology to add a nucleic acid homopolymer such as polydeoxyadenine to a functional nucleic acid so as to enable formation of a complex of a functional nucleic acid and ß-1,3-glucan such as SPG has been reported (for example, see Patent Literature 1). Also, a technology that uses ß-1,3-glucan such as SPG as a gene carrier by taking advantage of the aforementioned characteristics of ß-1,3-glucan has been reported (for example, see Patent Literature 2). Moreover, a technology that adds a nucleic acid-binding functional group such as a cationic functional group, steroidal functional group, amino acid functional group, or an intercalating functional group to at least part of the 1,6-glucopyranoside branch of ß-1,3-glucan in order to enhance the affinity between ß-1,3-glucan such as SPG and a nucleic acid has been reported (for example, see Patent Literature 3).

On the other hand, an ASO to which a polydeoxyadenine chain has been added does not usually form a complex with SPG at a short hetero sequence (for example, a polynucleotide such as antisense DNA in which bases are randomly arranged) portion. This is because a long polydeoxyadenine chain in particular forms a stable complex. So far, the inventors have confirmed that adding phosphorothioated polydeoxyadenine to a phosphorothioated ASO allows a stable SPG complex (PS complex) to be formed (see Non Patent Literature 2). However, there is a concern that a side effect (cytotoxicity) attributable to phosphorothioate is brought about when the PS complex is used. Currently, a technology to inhibit such a side effect is not yet found.

With such conventional art as background, the development of a method for efficiently obtaining a stable complex of SPG and an antisense DNA as well as a technology to inhibit the side effect of the PS complex is desired.

### [Citation List]

### [Non Patent Literature]

[NPL 1] P. S. Zamecnik et al., Proc Natl Acad Sci USA 75 (1978) 280-4 [NPL 2] Bull. Chem. Soc. Jpn., 77,1101-1110 (2004)

### [Patent Literature]

[PTL 1] WO 2007/058323
[PTL 2] WO 01/34207
[PTL 3] WO 02/072152

### [Summary of Invention]

### [Technical Problem]

A primary object of the present invention is to provide a highly stable, low-cytotoxicity nucleic acid-polysaccharide complex of an antisense DNA and a polysaccharide with a ß-1,3-glucan skeleton.

### [Solution to Problem]

Having conducted diligent research to solve the foregoing problems, the inventors found that adjusting the extent of S modification (phosphorothioation) of the polysaccharide binding site (i.e., polydeoxyadenine portion) in a nucleic acid-polysaccharide complex makes it possible to efficiently obtain a nucleic acid-polysaccharide complex that has superior stability and sufficient antisense activity to inhibit expression of a target gene. Moreover, the inventors also found that such a nucleic acid-polysaccharide complex exhibits reduced cytotoxicity. Based on these findings, the inventors made further improvements and arrived at the present invention.
That is, the present invention has the following aspects:
Item. 1 A nucleic acid-polysaccharide complex of an antisense DNA having polydeoxyadenine (dA) as a polysaccharide binding site and a polysaccharide having a polysaccharide ß-1,3-glucan skeleton, at least part of phosphodiester links of the polydeoxyadenine being phosphorothioated,
   provided that the phosphodiester links of the antisense DNA and the polydeoxyadenine are not entirely phosphorothioated.
Item. 2 The nucleic acid-polysaccharide complex according to Item 1, wherein the polysaccharide having ß-1,3-glucan skeleton is schizophyllan.
Item. 3 The nucleic acid-polysaccharide complex according to Item 1 or 2, wherein at least 20% of the phosphodiester links of the polydeoxyadenine are phosphorothioated.
Item. 4 The nucleic acid-polysaccharide complex according to any of Items 1 to 3,
wherein 25 to 90% of the phosphodiester links of the polydeoxyadenine are phosphorothioated.
Item. 5 The nucleic acid-polysaccharide complex according to any of Items 1 to 4, used to inhibit expression of a target gene.
Item. 6 A pharmaceutical composition including a nucleic acid-polysaccharide complex of any of Items 1 to 5 and a pharmaceutically acceptable carrier.
Item. 7 A method for producing a nucleic acid-polysaccharide complex, including a step of binding an antisense DNA having polydeoxyadenine (dA) as a polysaccharide binding site and a polysaccharide having ß-1,3-glucan skeleton via the polysaccharide binding site,
   in the nucleic acid-polysaccharide complex, at least part of phosphodiester links of the polysaccharide binding site being phosphorothioated, but the phosphodiester links of the antisense DNA and the polydeoxyadenine being not entirely phosphorothioated.
Item. 8 A method for introducing a nucleic acid-polysaccharide complex into a target cell, including a step of bringing a nucleic acid-polysaccharide complex of any of Items 1 to 5 into contact with the target cell.
Item. 9 A method for inhibiting expression of a target gene, including the step of bringing a nucleic acid-polysaccharide complex of any of Items 1 to 5 into contact with a cell having the target gene.
Item. 10 A method for stabilizing a nucleic acid-polysaccharide complex, including the step of binding an antisense DNA having polydeoxyadenine (dA) as a polysaccharide binding site and a polysaccharide having ß-1,3-glucan skeleton via the polysaccharide binding site,
   20 to 90% of phosphodiester links of the polydeoxyadenine being phosphorothioated.
Item. 11 A method for reducing cytotoxicity of a nucleic acid-polysaccharide complex, including the step of binding an antisense DNA having polydeoxyadenine (dA) as a polysaccharide binding site and a polysaccharide having ß-1,3-glucan skeleton via the polysaccharide binding site,
   20 to 90% of phosphodiester links of the polydeoxyadenine being phosphorothioated.
Item. 12 Use of a nucleic acid-polysaccharide complex for the manufacture of an inhibitor of expression of a target gene,
   the nucleic acid-polysaccharide complex being a complex of an antisense DNA having polydeoxyadenine (dA) as a polysaccharide binding site and a polysaccharide having ß-1,3-glucan skeleton, at least part of phosphodiester links of the polydeoxyadenine being phosphorothioated, but the phosphodiester links of the antisense DNA and the polydeoxyadenine being not entirely phosphorothioated.

### [Effect of Invention]

The nucleic acid-polysaccharide complex composed of an antisense DNA and a polysaccharide with a ß-1,3-glucan skeleton of the present invention exhibits superior stability and reduced cytotoxicity, and therefore can be safely used also in antisense therapy. Also, the nucleic acid-polysaccharide complex of the present invention can demonstrate a superior antisense effect.

### [Brief Description of Drawings]

FIG. 1(A) shows migration patterns of complex samples (c) and naked (n) (dA)₂₀, (dA)₃₀ and (dA)₄₀ samples in polyacrylamide gel electrophoresis.
FIG. 1(B) shows migration patterns ofAS-(dA)₂₀, AS-(dA)₃₀ andAS-(dA)₄₀ samples in polyacrylamide gel electrophoresis. Note that the a naked (n) (dA)₂₀ sample refers to dA that is not complexed with SPG.
FIG. 2(A) shows migration patterns of complex samples (c) and naked (n) (dA)₄₀ samples having different PS contents in polyacrylamide gel electrophoresis. FIG. 2(B) shows gel permeation chromatograms of a complex composed of SPG and AS-ODN with PS and a complex composed of SPG and AS-ODN with PO. For the chromatogram, UV absorbance at 260 nm was detected.
FIG. 3-1 shows the PS content dependencies of the CD spectra of naked (dA)₄₀ samples (FIG. 3-1(A)) and (dA)₄₀ complexes (FIG. 3-1(B)) measured at 20°C.
FIG. 3-2(C) shows the temperature dependencies of the CD spectra of complexes at 282 nm. FIG. 3-2(D) shows Tm of (dA)₄₀ samples having different PS contents.
FIG. 4 shows a comparison of the migration patterns in gel electrophoresis of complexes composed of SPG and ASO-(dA)₄₀ with different PS contents. In the figure, n refers to a naked ASO and c refers to a complex formed with SPG.
FIG. 5 shows a comparison of LPS-induced TNF-α secretion after treatment withASO/SPG complexes. Results are shown as mean ± SD (n = 3), * P < 0.05, ** P < 0.01

### [Description of Embodiments]

The nucleic acid-polysaccharide complex of the present invention has a triple helix conformation composed of two strands of a polysaccharide with a ß-1,3-glucan skeleton and a single strand of an antisense DNA with a base sequence that is complementary to the target sequence. Specifically, in the process in which the polysaccharide with a ß-1,3-glucan skeleton, which is a polysaccharide having a triple helix conformation, is dissolved in an organic solvent or an aqueous alkaline solution to form a solution of single chains and this solvent is then brought back to the original neutral fluid, presence of poly(dA) that is longer than a specific length causes a complex having two polysaccharide chains per one polynucleotide chain to be formed in a length proportion of one polysaccharide main chain glucose to one nucleic acid base.

The antisense DNA constituting the nucleic acid-polysaccharide complex of the present invention has a polydeoxyadenine (dA) tail at its terminal. The antisense DNA is a single strand DNA that can inhibit expression of a target gene by inhibiting transcription of the target gene to mRNA. The number of bases that constitute the antisense DNA is not particularly limited, and may be usually 10 to 35, preferably 15 to 30, and more preferably 16 to 24. The target gene that is a subject of expression inhibition by the antisense DNA is not particularly limited, and can be suitably determined according to, for example, the purpose of treatment. Also, the base sequence of the antisense DNA can be designed by a known technique according to, for example, the target gene. Also, the dA tail that constitutes the nucleic acid-polysaccharide complex of the present invention functions as a polysaccharide binding site. The number of deoxyadenines that constitute the dA tail is not particularly limited as far as the complex can be formed with a polysaccharide with a ß-1,3-glucan skeleton, which will be described below. For example, the number may be 10 to 100, preferably 20 to 100, more preferably 20 to 80, and even more preferably 40 to 60.

Modifying the antisense DNA to include the dA tail allows the complex to be favorably formed with a polysaccharide with a ß-1,3-glucan skeleton, and the nucleic acid-polysaccharide complex obtained in this manner has a high level of resistance to degrading enzymes.

Specifically, in order to directly bind the dA tail to the 5' end of the antisense DNA strand, the 5' carbon atom of the ribonucleotide at the 5' end of the antisense DNA strand may be ester-linked with the phosphate residue that is ester-linked with the 3' carbon atom at the 3' end of the dA tail. Also, in order to directly bind the dA tail to the 3' end of the antisense DNA strand, the phosphate residue that is bound to the 3' end ribonucleotide of the antisense DNA strand and the 5' end of the dA tail may be ester-linked.
Also, the antisense DNA may be one that has been variously modified by methods that are usually used. For example, for stabilization, it is possible to use an S-oligo that has been phosphorothioated (S-modified) or a nucleic acid in which the 2' position of the sugar has been modified with fluorine, a hydroxymethyl group, or the like. However, a complex in which the phosphodiester link portion between the dA tail and the antisense DNA is entirely S-modified is not encompassed within the present invention. The extent of S modification on the antisense portion varies depending on the purpose of treatment, and can be suitably adjusted. For example, in the case where the enzyme resistance to nuclease is needed, a complex having an extent of S modification closer to 100% is preferable, and in the case where there is a concern of a side reaction resulting from S modification, a complex that is similar to a naturally occurring type (i.e., an extent of S modification closer to 0%) is preferable.

In the present invention, the phosphodiester links of the polysaccharide binding site of polydeoxyadenine (dA) are phosphorothioated (S-modified) so as to satisfy a specific range, thus making it possible to enhance the yield and stability and reduce the cytotoxicity of the nucleic acid-polysaccharide complex of the present invention.

S modification on polydeoxyadenine can be performed according to a known method. The distribution of S modification in the dA tail portion is not particularly limited, and the desired S modification may be performed on any location. Here, in order to enhance the yield of the nucleic acid-polysaccharide complex, the extent of S modification is about 20 to 90%, preferably about 30 to 70%, and more preferably about 50 to 70%. Note that, herein, the extent of S modification of the dA tail portion refers to the proportion (%) of S-modified phosphodiester links relative to the total phosphodiester links in the dA tail. Also, the S-modified phosphodiester link denotes a binding structure in which one oxygen atom of the phosphate residue of a phosphodiester link is replaced by a sulfur atom.

Also, in order to enhance the stability of the nucleic acid-polysaccharide complex, the extent of S modification is about 20 to 90%, preferably about 30 to 70%, and more preferably about 50 to 70%, with the total length of the dA tail being 100%.

Moreover, in order to reduce the cytotoxicity of the nucleic acid-polysaccharide complex, the extent of S modification is about 20 to 90% and preferably about 30 to 70%, with the total length of the dA tail being 100%.

In the present invention, in order to demonstrate all of the high yield, stability, and cytotoxicity reducing effects, it is desirable that the extent of S modification on polydeoxyadenine is 20% or greater, preferably about 25 to 90%, and more preferably about 30 to 70%.

The nucleic acid-polysaccharide complex of the present invention contains a polysaccharide with a ß-1,3-glucan skeleton as a portion that is responsible for the desired function other than the antisense effect. The polysaccharide with a ß-1,3-glucan skeleton is delivered into a cell through a signal that is induced when binding to dectin-1, which is an aforementioned receptor present on the antigen presenting cell surface.

ß-1,3-Glucan is a polysaccharide in which glucose monomers are bound to each other by ß1→3 glycoside linkage, and there are a variety of known ß-1,3-glucans in which the proportion of the number of side chain glucose residues to the number of main chain glucose residues (hereinafter referred to as a side chain content) is different. For the polysaccharide used in the present invention, from the viewpoint of making it easy to form a complex with the aforementioned single strand polydeoxyadenine and further enhancing the introducibility of the nucleic acid-polysaccharide complex of the present invention into a cell, a polysaccharide with a ß-1,3-glucan skeleton having a relatively high side chain content is suitable.
Specific examples of the polysaccharide with a ß-1,3-glucan skeleton used in the present invention (hereinafter sometimes simply referred to as the "foregoing polysaccharide") include schizophyllan, curdlan, lentinan, pachyman, grifolan, scleroglucan, and the like. Among these, because schizophyllan allows the cell introducibility and enzyme degradation resistance to be more significantly enhanced, schizophyllan is a suitable polysaccharide in the present invention. In the case where schizophyllan is used for the polysaccharide, it is possible to introduce a functional group (such as an amino group, amino acid group, peptide group, or cholesterol group) that has affinity to a cellular receptor through chemical conversion of the side chain glucose residue of schizophyllan.

The molecular weight of the foregoing polysaccharide used in the nucleic acid-polysaccharide complex of the present invention is not particularly limited, and may be suitably set according to the polysaccharide used, the chain length of the above-described dA tail, and the like. Specifically, the molecular weight of the foregoing polysaccharide is usually, for example, 25000 to 2500000 and preferably 25000 to 150000.
A functional molecule may be directly bound to the foregoing polysaccharide or may be bound via a linker. Accordingly, use of the foregoing polysaccharide to which a functional molecule has been bound (a modified polysaccharide with a ß-1,3-glucan skeleton) makes it possible to impart a useful function that is attributable to the functional molecule to the nucleic acid-polysaccharide complex of the present invention. A known linker can be used, and an example may be PEG or the like.

Specific examples of the functional molecule include peptides, proteins, sugars, amino acids, DNAs, RNAs, low-molecular organic or inorganic materials, cholesterols, dendrimers, lipids, macromolecular materials, and the like.

Examples of the peptides include peptides composed of 3 to 40, preferably 6 to 30, and more preferably 8 to 25 amino acids. Specific examples include cellular membrane permeable peptides (such as octaarginine (R8) and penetrating, nuclear localization signal peptide sequences (such as HIV-1 Tat and SV40 T antigen), nuclear export signal peptides (such as HIV-1 Rev and MAPKK), and cellular membrane fusion peptides (such as gp41 and viral fusion peptides). In particular, R8 is suitably used in the present invention. R8 has an effect to facilitate the introduction into a cell, and use of an R8-bound polysaccharide is advantageous because the nucleic acid-polysaccharide complex can be more efficiently introduced into a cell.

For the proteins, proteins present in living bodies, proteins that have pharmacological actions, proteins that have molecular recognition actions, and like proteins can be used. Examples of the proteins include exportin/importin proteins, fibronectin, avidin, antibodies, and the like.

Examples of the sugars include monosaccharides such as glucose, galactose, glucosamine, and galactosamine; oligosaccharides or polysaccharides in which such monosaccharides are used in any combination; and the like.

Examples of the low-molecular organic or inorganic materials include cationic molecules such as spermine and spermidine; fluorescent substances such as FITC, Alexa, Cy3, and Cy5; biotin; quantum dots; fine gold particles; and the like.

Examples of the dendrimers include polyamideamine dendrimers and the like.

Examples of the lipids include linoleic acid,
1,2-dioleoyl-sn-glycero-3-phosphoethanolamine (DOPE), and the like.

Examples of the macromolecular materials include polyethylene glycol, polyethyleneimine, and the like.

Among the aforementioned functional molecules, peptides, cationic molecules, and polyethylene glycol are preferable, and peptides are more preferable. Also, among the functional molecules, molecules that have cell membrane permeability (cell membrane permeable molecules) and molecules that can impart a degrading enzyme resistance to the antisense DNA are suitable. Among the functional molecules above, the cell membrane permeable molecule may be a peptide or the like, and the molecule that can impart a degrading enzyme resistance to the antisense DNA may be polyethylene glycol. Modified polysaccharides having such a functional molecule are encompassed within the polysaccharide with a ß-1,3-glucan skeleton in the present invention.

The nucleic acid-polysaccharide complex of the present invention is formed by complexation of the above-described antisense DNA with a dA tail and the foregoing polysaccharide. The complex conformation of the antisense DNA and the foregoing polysaccharide is not particularly limited, and usually a triple helix conformation formed by complexation of one strand of the antisense DNA and two strands of the foregoing polysaccharide is suitable. Specifically, this triple helix conformation can be formed by the following method. The foregoing polysaccharide adopts a triple helix conformation in a natural environment or in water. This polysaccharide is dissolved in a polar solvent such as dimethylsulfoxide (DMSO) to give single strands, then the antisense DNA with a dA tail is added, the solvent is replaced with water (regeneration process), and thereby a triple helical complex conformation (association structure) composed of one strand of the antisense DNA and two strands of the foregoing polysaccharide is formed. It seems that such complex formation of a polynucleotide and a polysaccharide is achieved through hydrogen bonding and a hydrophobic interaction.

The nucleic acid-polysaccharide complex of the present invention having such a conformation can be prepared according to a known method. A specific example may be a production method that has the following steps (1) to (3): (1) a polynucleotide-bound antisense DNA to which polydeoxyadenine is bound is prepared according to a known method, (2) separately, the foregoing polysaccharide is provided, or the foregoing polysaccharide to which a functional molecule is bound directly or via a linker is prepared, and (3) a complex is then formed using the polydeoxyadenine that is bound to the antisense DNA and the foregoing polysaccharide.

That is, the present invention also provides a method for producing a nucleic acid-polysaccharide complex in which an antisense DNA having polydeoxyadenine (dA) as a polysaccharide binding site and a polysaccharide with a ß-1,3-glucan skeleton are bound via the polysaccharide binding site. Here, at least part of the polydeoxyadenine portion (dA tail portion) is S-modified, and the extent of S modification is as stated above.

In the complex formed in the step (3) of the aforementioned method, the ratio of the molecules of the antisense DNA with a dA tail to the molecules of ß-1,3-glucan is suitably set according to, for example, the number of bases of the antisense strand, the number of adenines of the dA tail added thereto, and the molecular weight of ß-1,3-glucan. Although it is not possible to uniquely specify a preferable ratio of molecules, the ratio of molecules (in mole) between ß-1,3-glucan and the antisense DNA with a dA tail is usually about 1 : 2 to 1 : 30. In the case where the efficacy can be more effectively demonstrated with a complex having a large amount of the antisense strand relative to one molecule of ß-1,3-glucan, it is preferable to form a complex with ß-1,3-glucan having a molecular weight of a few tens of thousands or greater, and a ratio of molecules (in mole) of 1: 8 to 1 : 20 is more preferable. On the other hand, in the case where the complex in a small particulate form is effective, a ratio of molecules (in mole) of 1: 2 to 1: 5 is more preferable. Subjecting the antisense DNA and schizophyllan to complex forming conditions in such a molar ratio enables these materials to efficiently interact with each other, and it is thus possible to enhance the production efficiency of the nucleic acid-polysaccharide complex of the present invention.

The stoichiometric ratio of the complex is 2 :1 in terms of the molar ratio of the glucose sugar that is a repeating unit of the ß-1,3-glucan skeleton to the base of polydeoxyadenine. Usually, because polysaccharide and polydeoxyadenine are macromolecules, it is not possible to specify the stoichiometric ratio of the complex by way of the moles of polysaccharide and polydeoxyadenine. Experimentally, it is convenient to use the weight ratio or the ratio of molecules between the sugar and the nucleic acid to be reacted. Therefore, although the stoichiometric ratio of the complex is 2 : 1, in the case where the nucleic acid needs to be strongly protected, the sugar may be used in a greater amount, and in order to strongly exhibit the effect of the antisense nucleic acid, the nucleic acid may be used in a greater amount.

The nucleic acid-polysaccharide complex of the present invention once introduced into a cell can inhibit expression of a target gene in the cell, and can thus be used as a pharmaceutical composition intended to inhibit expression of the target gene. The pharmaceutical composition can be prepared by suitably combining the nucleic acid-polysaccharide complex of the present invention as an active ingredient with a pharmaceutically acceptable carrier. Examples of such a carrier include aqueous carriers such as purified water, sugar-containing aqueous solutions, buffers, physiological saline, and RNase-free water; excipients; and the like.

The dosage of the nucleic acid-polysaccharide complex of the present invention is not particularly limited as long as the desired gene inhibiting action can be demonstrated. It is desirable that the amount of the nucleic acid administered in one dose is 0.001 mg to 10 mg per kg body weight, and it can be suitably set according to the symptom of the administration subject, administration route, and the like.

Also, the dosage form of the pharmaceutical composition, the administration method, and the like may be those that are known in regard to pharmaceutical products, and can be suitably set according to the antisense DNA, use, disease site, and the like.

Moreover, the present invention provides a method for introducing a nucleic acid-polysaccharide complex into a target cell. The amount of the nucleic acid-polysaccharide complex of the present invention introduced into a cell and the introduction method may be the same as those with conventional antisense DNAs, and the nucleic acid-polysaccharide complex in an amount effective to inhibit expression of the target gene may be brought into contact with a cell that has the target gene to allow the cell to uptake the nucleic acid-polysaccharide complex. Note that the nucleic acid-polysaccharide complex of the present invention, even when used singly, demonstrates strong ability to migrate into a cell, and it is thus possible to introduce the nucleic acid-polysaccharide complex into a cell without a conventional gene transfection reagent that has been used to introduce an antisense DNA into a cell, or with a conventional gene transfection reagent in a reduced amount. Note that the inhibition of expression of the target gene by the nucleic acid-polysaccharide complex of the present invention may be carried out *in vivo* as well as *in vitro* or *ex vivo.*

Moreover, adjusting the extent of S modification of the polydeoxyadenine (dA) tail portion as described above makes it possible to obtain a highly stable nucleic acid-polysaccharide complex and a nucleic acid-polysaccharide complex with reduced cytotoxicity. That is, the present invention provides a method for stabilizing a nucleic acid-polysaccharide complex, wherein 20 to 90%, preferably 30 to 70%, and more preferably 50 to 70% of the phosphodiester links of the dA tail portion are phosphorothioated, and a method for reducing the cytotoxicity of a nucleic acid-polysaccharide complex, wherein 20 to 90% and preferably 30 to 70% of the phosphodiester links of the dA tail portion are phosphorothioated.

Also, the present invention provides use of the above-described nucleic acid-polysaccharide complex to inhibit expression of a target gene in a cell. Moreover, the present invention provides a method for inhibiting expression of a target gene using the nucleic acid-polysaccharide complex. With regard to this use and method, the nucleic acid-polysaccharide complex and the manner of using the complex are as described above.

### [Examples]

The present invention shall be described in more detail below by way of examples, but the present invention is not limited thereto. Note that in the examples, schizophyllan may be referred to as "SPG", and polydeoxyadenine may be referred to as "(dA)ₓ". Also, an antisense DNA may be referred to as "AS".

### 1. Test materials and test methods

### (1.1) Test materials

*Schizophyllum commune-derived* SPG (M_{w} =1.5 × 10⁵: measured by multi-angle light scattering analysis and gel permeation chromatography in combination) was provided from Mitsui Sugar Co., Ltd. A phosphorothioated antisense DNA sample was synthesized by Fasmac Co., Ltd., and purified by high performance liquid chromatography

### (1.2) Preparation of phosphorothioated antisense DNA complex with SPG

SPG was dissolved in a 0.25 N aqueous NaOH solution for at least two days to dissociate the triple helical strands of SPG into single strands. A suitable amount of the SPG solution, oligonucleotide (in water), and a phosphate buffer (330 mM NaH₂PO₄, pH = 4.7) were mixed. Then, the mixture (oligonucleotide 60 µM, pH = 7.4) was left to stand at 4°C overnight. The molar ratio ([SPG] / [ODN]) expressed in the ratio of SPG molecules to oligonucleotide (ODN) molecules was adjusted so as to be 0.27.

### (1.3) Polyacrylamide gel electrophoresis (PAGE)

Oligonucleotide (20 ng) or the complex of oligonucleotide and SPG was separated over 1 hour using 15% acrylamide gel at 100V. Gel was stained with SYBR Gold (manufactured by Invitrogen) and then subjected to a PharosFX (manufactured by Bio-Rad Laboratories) to give a fluorescence image.

### (1.4) Gel permeation chromatography (GPC)

GPC was carried out at a flow rate of 0.8 ml/min with OHpak SB802.5 and OHpak 806 columns using a Showa Denko DU-H2130 pump. A 0.5 M KCL-containing 0.1 M phosphate buffer solution (pH 7.4) was used for a mobile phase. The eluate was examined by a multi-angle light scattering (LS) detector (DAWN-HELEOS, manufactured by Wyatt Technology Corporation), a UV detector (SPD-10A, manufactured by Shimadzu Corporation), and a refractive index (RI) detector (RI-71S, Showa Denko K.K.). The RI and LS signals were used to calculate the weight average molecular weight (Mw) according to the instruction manual of the DAWN-HELEOS. The refractive index increment (a value of dn / dc) was 0.157 ml/g.

### (1.5) Circular dichroism spectroscopy

The circularly polarized light in a 240 to 320 nm region was measured by a Jasco J-802 circular dichroism dispersion analyzer (manufactured by Jasco Corporation) at 20 to 80°C using a 1 cm quartz cell with a thermostat.

### (1.6) Animal testing

Animal testing was carried out according to the guideline by the Kyushu Institute of Technology animal experiment committee. A thioglycolate-induced macrophage was isolated from a C57BL/6 mouse (obtained from Kyudo Co., Ltd.) according to a standard procedure. In brief, 3 mL of a 3% thioglycollate medium was intraperitoneally injected into a mouse, then peritoneal lavage was performed with RPMI 1640 (6 mL) 3 days later, and the peritoneal macrophage was collected. The lavage fluid was retained and centrifuged for 5 minutes at 100× g. The first-generation macrophage was cultured in a RPMI 1640 medium supplemented with 10% FCS, 100 U/ml penicillin, and 0.1 mg/ml streptomycin under 37°C and 5% CO₂ conditions.

### (1.7) Cytokine assay

The macrophage obtained in (1.6) above was seeded onto a 94 well plate in a concentration of 5 × 10⁴ cells/well and left to stand for 24 hours. An ODN/SPG complex or ODN (0.5 µM) was added to the cells, incubation was carried out at 37°C for 30 minutes, and then incubation was carried out at 37°C for 5 hours in an LPS (10 ng/ml)-containing fresh medium. The level of TNF-α released into the supernatant was measured using a murine TNF-α ELISA Development kit (manufactured by PeproTech).

### 2. Results and discussions

### (2.1) Complex formation between SPG and (dA)ₓ

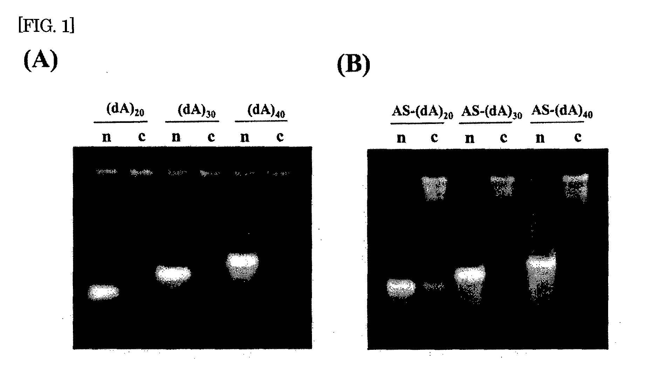

FIG. 1A shows the sequence length dependency in complex formation of phosphorothioated (dA)ₓ (PS-(dA)ₓ) detected by polyacrylamide gel electrophoresis (PAGE). With dAₓ having x > 30, no free (dA)ₓ was detected after formation of a complex with SPG. On the other hand, a small amount of free (dA)₂₀ was detected when x = 20. As can be understood by comparing the second lanes in FIGS 1A and 1B, it was shown that in the case where a hetero sequence that biologically actually exists, such as 20 mer antisense TNF-α, is bound to (dA)₂₀, the yield is smaller than that of (dA)₂₀ alone. Also, as the terminal became longer, i.e., with theAS-(dA)₃₀ and AS-(dA)₄₀, free (dA)ₓ was barely found. These results show that a long dA terminal contributes to a high yield, and the bound hetero sequence lowers the yield. It is understood that this effect of the terminal length on the yield is due to the stabilization of the complex resulting from a cooperative macromolecular effect.

The inventors prepared six (dA)₄₀ samples having different PS contents (extents of S modification) (PS contents: 0/39 PS, 8/39 PS, 13/39 PS, 20/39 PS, 26/39 PS, 39/39 PS). These six (dA)₄₀ samples are shown in Table 1 below. Note that in the (dA)₄₀ samples, various dA tails were directly bound to the 3' end of antisense TNF-α (aTNF).

**[Table 1]**

| (dA)₄₀ sample | DNA sequence | Extent of S modification | Yield | |
|---|---|---|---|---|
| 0/39-aTNF | AACCCATCGGCTGGCACCAC-A₄₀ | 0% | 70% | SEQ. ID. NO. 1 |
| 8/39-aTNF | AACCCATCGGCTGGCACCAC-(AsAAAA)₈ | 20.5% | 90% | SEQ. ID. NO. 2 |
| 13/39-aTNF | AACCCATCGGCTGGCACCAC-<AsAA)₁₃A | 33.3% | 100% | SEQ. ID. NO. 3 |
| 20/39-aTNF | AACCCATCGGCTGGCACCAC-(AsA)₂₀ | 51.3% | 100% | SEQ. ID. NO. 4 |
| 26/39-aTNF | AACCCATCGGCTGGCACCAC-(AsAsA)₁₃A | 66.7% | 100% | SEQ. ID. NO. 5 |
| 39/39-aTNF | AACCCATCGGCTGGCACCAC-(As)₃₉A | 100% | 100% | SEQ. ID. NO. 6 |

| | | | | |
|---|---|---|---|---|
| * "s" in the DNA sequences denotes a phosphorothioate link. | | | | |

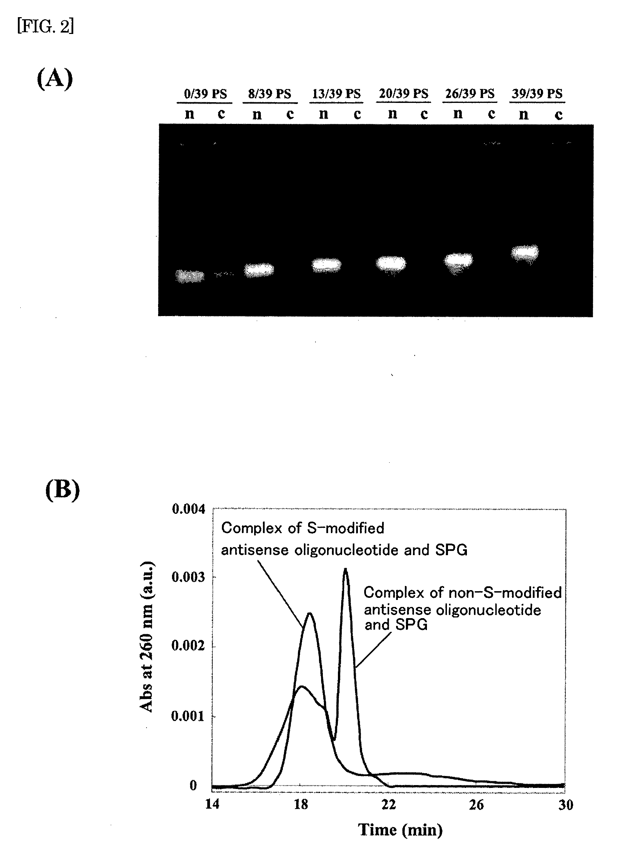

These complexes were prepared according to the description provided in the section titled "1. Test materials and test methods". As shown in the PAGE image of FIG. 2A, while no free (dA)ₓ was observed with the 13/39, 20/39, 26/39, and 39/39 PS complexes, a certain amount of free (dA)ₓ was observed with the 8/39 PS complex. The yield with 8/39 PS was 90%, and the yield with 0/39 PS was 70%. FIG. 2B compares gel permeation chromatograms (UV 260nm) after formation of complexes with 39/39 PS and 0/39 PS. It was measured that, from the peak areas (about 16 to 20 minutes) of the complexes and free (dA)ₓ (about 20 to 25 minutes), the yield with 39/39 PS was 90% and the yield with 0/39 PS was 60%, thus matching the results of PAGE. It was confirmed from the results provided above that approximately 30% phosphorothioate modification gives a yield of 100%, and PS (phosphorothioate) forms a more stable complex than PO (phosphodiester).

The relationship between phosphorylation and complex stability is interesting, and the inventors presumed that it relates to the molecular structure of the complex. Computational chemistry suggests that hydrogen bonds between the SPG backbone glucose and the base region is the driving force of complex formation, and in particular, hydrogen bonds cooperatively arranged along the SPG backbone glucose stabilize the complex (K. Miyoshi, et al., Biomacromolecules 6 (2005) 1540-6). The phosphate diester region binds to two adjacent riboses and determines the variable angle and stacking characteristics of the adjacent two (dA)₄₀ rings. Therefore, PS forms such hydrogen bonds and is thus likely to provide a more preferable steric configuration than PO. The spatial relationship of the two adjacent dA rings is fully reflected in circular dichroism (CD).

### (2.2) Thermal stability of complex

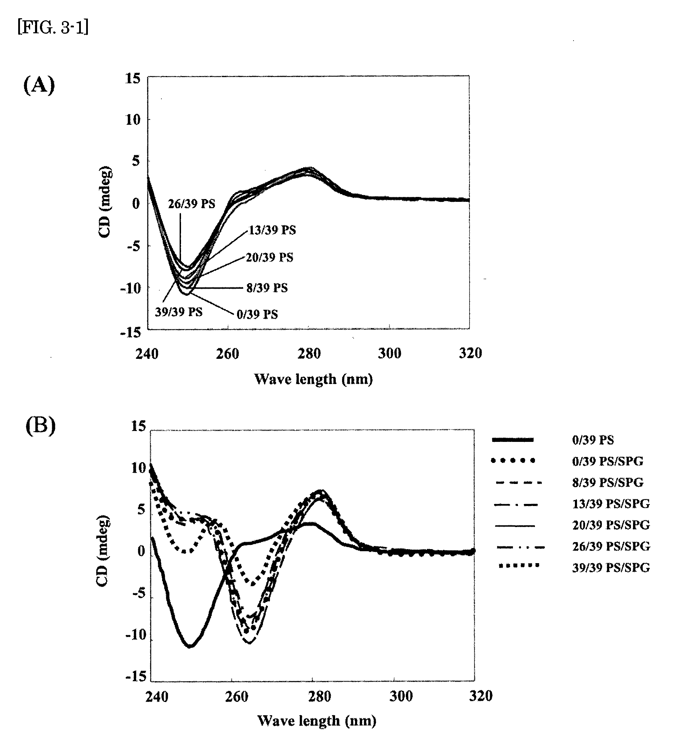

FIG. 3-1A shows CD spectra of all PS(dA)₄₀ and PO(dA)₄₀ samples at 20°C in the 240 to 320 nm region. The overall shapes of the spectra are mutually similar among all the samples, but with an increased PS content, the intensity of the negative peak at 248 nm was slightly weakened. This wavelength region relates to increased CD among base regions, and therefore similar CD spectra mean that all (dA)₄₀ samples have the same structure.

In SPG complex formation, CD spectra differ greatly as shown in FIG. 3-1B. In FIG. 3-1B, SPG complexes with PS-(dA)₄₀are compared with naked PO-(dA)₄₀, and as already reported, it is shown that dA adopts a new structure to form an SPG complex (J. Kurreck, Eur J Biochem 270 (2003) 1628-44 and S. M. Boye et al., Antisense Nucleic Acid Drug Dev 12 (2002) 95-102). Comparing the spectra of the complexes, four complexes 0/39 PS/SPG to 20/39 PS/SPG showed nearly the same spectrum. On the other hand, with an S content increased to 39/39, the complex showed a spectrum different from those of other complexes. The negative peak of 39/39 PS was lower at 248 nm and higher at 264 nm.

As stated above, as the extent of S modification increased from PO to PS, the CD band around 250 nm decreased accordingly in FIG. 3-1A. This shows that the molecular configuration shifts from a natural form to a non-natural form as the S modification progresses. Side effects of synthesized nucleic acid medicines are thought to be caused by non-natural configurations. Therefore, the antisense oligonucleotide before complex formation exhibits the effect of a non-natural form as the extent of S modification increases. On the other hand, as shown in FIG. 3-1B, regarding the complexes, the CD band around 265 nm changes as the extent of S modification from PO to PS increases, but the change up to an S modification of 26/39 is extremely small, and the change is nearly the same when the S modification is at least 0/39 to 26/39. That is, it seems that in the PS complexes having an S modification of up to 26/39 retain a natural configuration, and a side effect is not likely to appear.

The above results also qualitatively match the aforementioned presumption that PS gives a more preferable structure than PO. However, these are qualitatively different from the results of CD and PAGE. That is, although 33% S modification (13/39 PS) was sufficient to achieve 100% yield, 66% S modification (26/39 PS) or greater was needed to change CD.

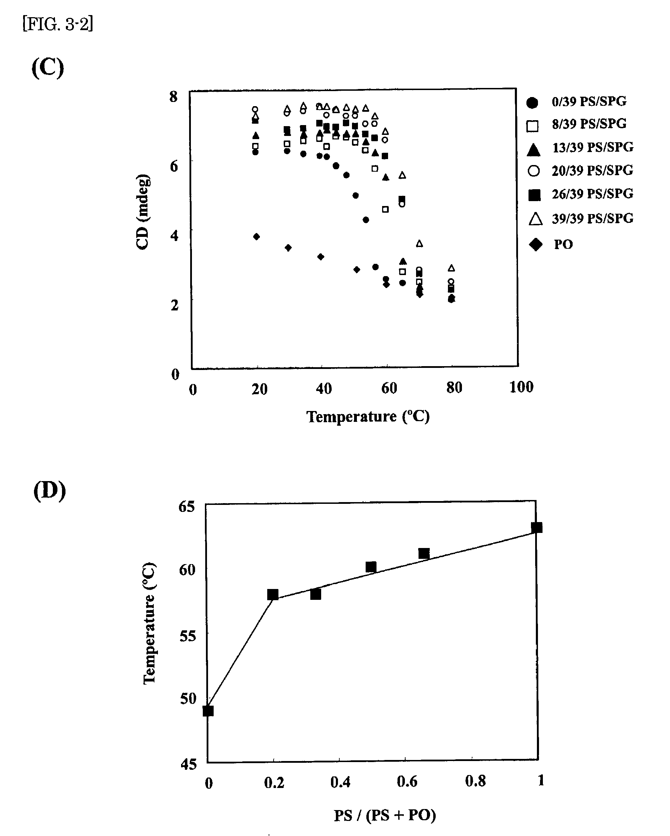

In FIG. 3-2(C), the CD intensity at 282 nm is plotted against temperature. As described above, the CD intensities of the complexes are greater than that of the naked sample at low temperatures. The CD intensities of the complexes decreased at temperatures within a specific range, and overlapped with the intensity of the corresponding (dA)₄₀ sample. Based on this transition, the dissociation temperature Tm (melting temperature) is plotted in FIG. 3-2D. As the PS content increased from 0/39 to 8/39, Tm increased from 49°C to 59°C, but a further increase of the PS content did not largely increased Tm. It was confirmed from these results that modification to have a PS backbone leads to a complex that is more stable than a complex with a PO backbone, and only 20% modification (S modification) is sufficient to give the same Tm increase as that brought about by 100% modification.

### (2.3) Gene silencing by complex

The inventors prepared five antisense oligonucleotide (ASO) samples, i.e., samples with different S contents in which the (dA)₄₀ tail was directly bound to the 3' end of a PO TNF-α antisense sequence and a completely phosphorothioated sample. Preparation of the samples was carried out according to the method described in sections (1.1) and (1.2) above. The extent of S modification and the yield of each sample are shown in Table 2 below.

**[Table 2]**

| ASO sample | Extent of S modification | Yield | |
|---|---|---|---|
| 0/39-aTNF | 0% | 50% | SEQ. ID. NO. 1 |
| 8/39-aTNF | 20.5% | 60% | SEQ. ID. NO. 2 |
| 13/39-aTNF | 33.3% | 100% | SEQ. ID. NO. 3 |
| 20/39-aTNF | 51.3% | 100% | SEQ. ID. NO. 4 |
| 26/39-aTNF | 66.7% | 100% | SEQ. ID. NO. 5 |
| 39/39-aTNF | 100% | 100% | SEQ. ID. NO. 6 |

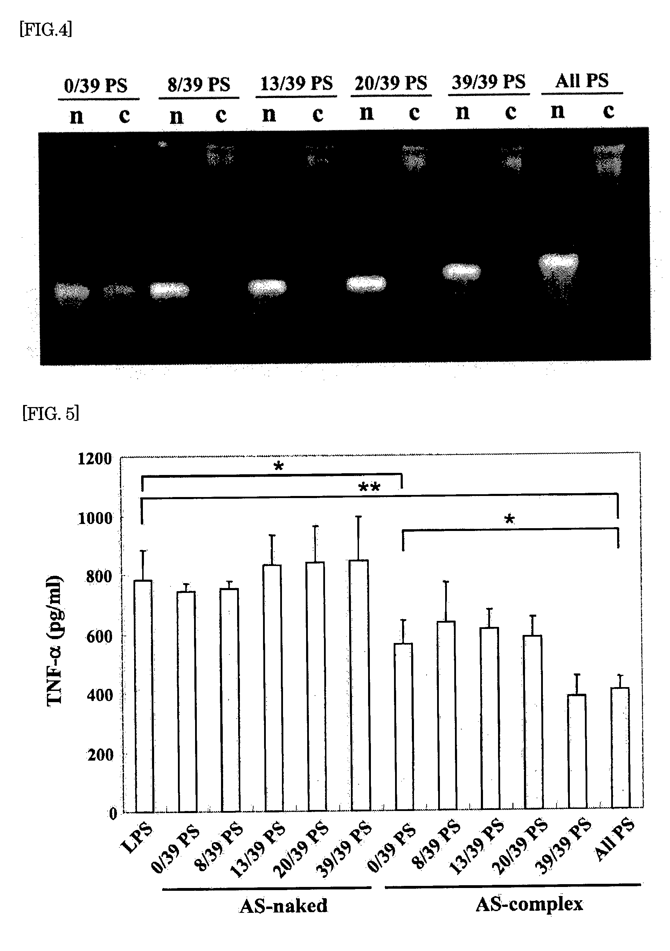

The PAGE image of all complexes is shown in FIG. 4. Regarding the AS-0/39 complex, a large amount of free antisense oligonucleotide was present, and the yield (extent of complex formation) was 50%. This value is smaller than that of the 0/39 PS (dA₄₀) tail itself. As the S content increased, the band of free antisense oligonucleotide disappeared, showing that the extent of complex formation increased. The AS-13/39 complex did not show the band of free antisense oligonucleotide and showed the same result as that of the (dA)₄₀ itself.

These inventors examined as described below the TNF-α gene silencing effect using the ASO samples prepared above.

An SPG complex is taken up into a cell via dectin-1 that is a primary receptor that recognizes ß-1,3-glucan on various antigen presenting cells (APCs) including macrophages and dendritic cells. A thioglycolate-induced peritoneal macrophage (thio-macrophage) was prepared, an ODN/SPG complex was administered, and then LPS stimulation was given to secrete TNF-α. FIG. 5 shows a graph in which the amount of secreted TNF-α is plotted against the PS content. None of the naked antisense oligonucleotides reduced the secretion of TNF-α. On the other hand, SPG complexes showed TNF-α silencing. As the PS content increased, the silencing effect gradually strengthened. It seems that this is because PS complexes having a large extent of S modification in the polydeoxyadenine tail are more stable in a culture medium and thus are more easily taken up into a cell. Also, it seems that because the antisense inhibitory abilities between PS and PO were nearly the same or PS complexes were slightly more effective, the antisense portion does not necessarily need to have a PS backbone.

(2.4) It was shown above that the complex with phosphorothioated (dA)₄₀ is more thermally stable than the phosphate dieaster (dA)₄₀, and mere replacement of only 20 to 30% of oxygen in the phosphate diester region enhances stability to the same level as that of the completely phosphorothioated complex. A high dissociation temperature means thermodynamic stability, thus indicating that the nucleic acid-polysaccharide complex of the present invention stably exists in the living body. The antisense gene inhibitory abilities between PS and PO were at the same level or PS was slightly more effective. These findings are likely to lead to a new understanding of the interaction between ß-1,3-glucan and DNA and be useful for therapeutic PS oligonucleotide delivery with reduced side effects.

### [Sequence Listing Free Text]

SEQ ID NO. 1 is a base sequence in which A₄₀ is bound to the 3' end of a sequence (aTNF).
SEQ ID NO. 2 is a base sequence in which (AsAAAA)₈ is bound to the 3' end of a sequence (aTNF).
SEQ ID NO. 3 is a base sequence in which (AsAA)₁₃A is bound to the 3' end of a sequence (aTNF).
SEQ ID NO. 4 is a base sequence in which (AsA)₂₀ is bound to the 3' end of a sequence (aTNF).
SEQ ID NO. 5 is a base sequence in which (AsAsA)₁₃A is bound to the 3' end of a sequence (aTNF).
SEQ ID NO. 6 is a base sequence in which (As)₃₉A is bound to the 3' end of a sequence (aTNF).
## Claims

1. A nucleic acid-polysaccharide complex of an antisense DNA having polydeoxyadenine (dA) as a polysaccharide binding site and a polysaccharide having a polysaccharide ß-1,3-glucan skeleton,
at least part of phosphodiester links of the polydeoxyadenine being phosphorothioated,
provided that the phosphodiester links of the antisense DNA and the polydeoxyadenine are not entirely phosphorothioated.

2. The nucleic acid-polysaccharide complex according to claim 1, wherein the polysaccharide having ß-1,3-glucan skeleton is schizophyllan.

3. The nucleic acid-polysaccharide complex according to claim 1 or 2, wherein at least 20% of the phosphodiester links of the polydeoxyadenine are phosphorothioated.

4. The nucleic acid-polysaccharide complex according to any of claims 1 to 3, wherein 25 to 90% of the phosphodiester links of the polydeoxyadenine are phosphorothioated.

5. The nucleic acid-polysaccharide complex according to any of claims 1 to 4, which is used to inhibit expression of a target gene.

6. A pharmaceutical composition comprising a nucleic acid-polysaccharide complex of any of claims 1 to 5 and a pharmaceutically acceptable carrier.

7. A method for producing a nucleic acid-polysaccharide complex, comprising a step of binding an antisense DNA having polydeoxyadenine (dA) as a polysaccharide binding site and a polysaccharide having ß-1,3-glucan skeleton via the polysaccharide binding site,
in the nucleic acid-polysaccharide complex, at least part of phosphodiester links of the polysaccharide binding site being phosphorothioated, but the phosphodiester links of the antisense DNA and the polydeoxyadenine being not entirely phosphorothioated.

8. A method for introducing a nucleic acid-polysaccharide complex into a target cell, comprising a step of bringing a nucleic acid-polysaccharide complex of any of claims 1 to 5 into contact with the target cell.

9. A method for inhibiting expression of a target gene, comprising a step of bringing a nucleic acid-polysaccharide complex of any of claims 1 to 5 into contact with a cell having the target gene.

10. A method for stabilizing a nucleic acid-polysaccharide complex, comprising the step of binding an antisense DNA having polydeoxyadenine (dA) as a polysaccharide binding site and a polysaccharide having ß-1,3-glucan skeleton via the polysaccharide binding site,
20 to 90% of phosphodiester links of the polydeoxyadenine being phosphorothioated.

11. A method for reducing cytotoxicity of a nucleic acid-polysaccharide complex, comprising the step of binding an antisense DNA having polydeoxyadenine (dA) as a polysaccharide binding site and a polysaccharide having ß-1,3-glucan skeleton via the polysaccharide binding site,
20 to 90% of phosphodiester links of the polydeoxyadenine being phosphorothioated.

12. Use of a nucleic acid-polysaccharide complex for the manufacture of an inhibitor of expression of a target gene,
the nucleic acid-polysaccharide complex being a complex of an antisense DNA having polydeoxyadenine (dA) as a polysaccharide binding site and a polysaccharide having ß-1,3-glucan skeleton, at least part of phosphodiester links of the polydeoxyadenine being phosphorothioated, but the phosphodiester links of the antisense DNA and the polydeoxyadenine being not entirely phosphorothioated.


[Sequence Listing] 0007P.SEQs.txt

SEQUENCE LISTING

<110>  NAPAJEN PHARMA, INC.

<120>  Nucleic acid/polysaccharide complex

<130>  N 2881EU-kl

<140>  11 806 795.8
<141>  2011-07-12

<150>  JP2010-162039
<151>  2010-07-16

<160>  6

<170>  PatentIn version 3.1

<210>  1
<211>  20
<212>  DNA
<213>  Artificial

<220>
<223>  3' terminal of the sequence is linked with (A)40.

<400>  1
aacccatcgg ctggcaccac                                                    20


<210>  2
<211>  20
<212>  DNA
<213>  Artificial

<220>
<223>  3' terminal of the sequence is linked with (AsAAAA)8.

<400>  2
aacccatcgg ctggcaccac                                                    20


<210>  3
<211>  20
<212>  DNA
<213>  Artificial

<220>
<223>  3' terminal of the sequence is linked with  (AsAA)13-A.

<400>  3
aacccatcgg ctggcaccac                                                    20


<210>  4
<211>  20
<212>  DNA
<213>  Artificial

<220>
<223>  3' terminal of the sequence is linked with  (AsA)20.

<400>  4
aacccatcgg ctggcaccac                                                    20


<210>  5
<211>  20

<212>  DNA
<213>  Artificial

<220>
<223>  3' terminal of the sequence is linked with  (AsAsA)13-A.

<400>  5
aacccatcgg ctggcaccac                                                    20


<210>  6
<211>  20
<212>  DNA
<213>  Artificial

<220>
<223>  3' terminal of the sequence is linked with  (As)39-A.

<400>  6
aacccatcgg ctggcaccac                                                    20